Europäisches Patentamt

European Patent Office

Office européen des brevets

(11) Publication number: **0 267 624**
**A2**

## (12) EUROPEAN PATENT APPLICATION

(21) Application number: 87116795.3

(51) Int. Cl.⁴: **A61L 27/00** , //A61F2/28

(22) Date of filing: 13.11.87

(30) Priority: 14.11.86 JP 271059/86

(43) Date of publication of application:
18.05.88 Bulletin 88/20

(84) Designated Contracting States:
DE FR GB

(71) Applicant: ASAHI KOGAKU KOGYO
KABUSHIKI KAISHA
36-9, Maeno-cho 2-Chome Itabashi-ku
Tokyo 174(JP)

(72) Inventor: Ichitsuka, Takeshi
Asahi Kogaku Kogyo K.K. No. 36-9,
Maeno-cho 2-chom
Itabashi-ku Tokyo(JP)
Inventor: Yokoo, Akihiko
Asahi Kogaku Kogyo K.K. No. 36-9,
Maeno-cho 2-chom
Itabashi-ku Tokyo(JP)

(74) Representative: Hansen, Bernd, Dr.rer.nat. et
al
Hoffmann, Eitle & Partner Patentanwälte
Arabellastrasse 4
D-8000 München 81(DE)

(54) Porous calcium phosphate based bone prosthesis.

(57) A porous calcium phosphate based bone prosthesis having open pores with an average size of 0.01-2,000 µm and closed pores with an average size of 0.01-30 µm. The bone prosthesis has both good workability and an adequate degree of biocompatibility.

EP 0 267 624 A2

## POROUS CALCIUM PHOSPHATE BASED BONE PROSTHESIS

### FIELD OF THE INVENTION

The present invention relates to a porous calcium phosphate based bone prosthesis or bone filler that is useful in dentistry, oral surgery, orthopaedic surgery, otorhinolaryngology and other medical fields in which it is used for filling bone defects that result from bone fractures that have been caused by accident or from operations for various diseases such as osteoma and pyorrhea alveolaris.

### BACKGROUND OF THE INVENTION

Numerous research and development efforts have been directed to obtaining prosthetic materials that can be used to replace or fill portions of hard tissue of the human body where the tissue has been partially lost. The prosthetic materials developed thus far are roughly divided into two types, those which are made of natural bone and those which are formed of artificial materials.

The first type of prosthetic materials, those which are made of natural bone, include: autogeneous bone, which is taken from the bone at a normal site within the patient's body; homologous bone, which is taken from the bone at a normal site within the body of a living relative close to the patient or from frozen bone from a human cadavor; and heterologous bone, which is bone derived from a non-human (e.g., bovine or pig). Autogenous bone has the advantage that it is free from rejection by the body in which it is transplanted but the need to collect the bone tissue from a normal site within the patient's body imposes a great burden on the patient. In addition, it is not always possible to collect an adequate amount of autogenous bone tissue. The use of the bone of a relative close to the patient is comparatively free from the risk of rejection but the burden on the relative caused by performing the surgical operation to collect the necessary bone tissue still is great and the possibility of rejection is not completely avoidable. Use of frozen bone from a human cadaver or heterologous bone from a non-human source such as deproteinized calf bone involves great risk in that a strong reaction which can be fatal is caused by contact of this foreign material with the body tissues or fluids of the patient.

The second type of prosthetic materials, which are made of artificial materials, include metals such as cobalt-chrome alloys, titanium and stainless steel, ceramics such as alumina, zirconia, tri calcium phosphate, hydroxyapatite and calcium phosphate based glass, high-molecular weight materials such as silicone resins, and carbon.

Among these artificial prosthetic materials, ceramics are stable in the body and have a high degree of biocompatibility, so attempts have recently been made to use them as medical materials. Some of these efforts have been commercialized. Ceramics as medical materials, in particular as bone prosthesis, are classified into two types, bioinert and bioactive. "Bioinert" medical materials can be implanted in the body without causing any reaction with the surrounding hard tissues, and "bioactive" medical materials, when implanted in the body will establish a direct bond with new bone. Examples of bioinert materials are alumina and zirconia, and examples of bioactive materials are calcium phosphate based materials such as tri calcium phosphate, hydroxyapatite, and calcium phosphate based glass.

Sintered bioactive calcium phosphate based ceramics, in particular, hydroxyapatite, has been acclaimed for its high degree of biocompatibility and has been commercialized as artificial dental roots and other prosthetic products. Bone prosthesis have also been developed that are made of sintered porous hydroxyapatite in which enhanced circulation of body fluids occurs to cause calcification within the pores so that the prosthesis can combine with new bone. This porous calcium phosphate based structure is one of the bone prosthesis that is attracting the greatest interest of researchers and various sintered porous materials that are characterized by the form of the pores present have been proposed (see, for example, Unexamined Published Japanese Patent Application Nos. 16879/1985 and 21763/1985).

The form of the pores present in prior art sintered porous calcium phosphate based ceramic materials has been determined in such a way that the specific objectives of enhancing their compatibility with surrounding bones and their mechanical strength can be met and they may be highly credited with their performance in these respects. However, workability is another important factor for the sintered porous calcium phosphate based ceramic materials if they are to be used as bone prosthesis and, in this respect, the prior art products still leave room for improvement. Implantation of bone prosthesis involves fine trimming as an essential step both prior to and during surgical operation. Calcium phosphate based ceramics are easy to work as compared with other ceramics and can be ground or cut with diamond burs or disks, but difficulty does exist in trimming over large areas.

## SUMMARY OF THE INVENTION

An object, therefore, of the present invention is to provide a porous calcium phosphate based bone prosthesis that has a high degree of biocompatability and which can be worked more easily than the prior art products.

This and other objects have been attained by providing a porous calcium phosphate based bone prosthesis that has open pores with an average size of 0.01 -2,000 $\mu$m and closed pores with an average size of 0.01 - 30 $\mu$m.

## DETAILED DESCRIPTION OF THE INVENTION

The present inventors previously observed that encapsulation by fibrous tissue of a bone prosthesis implanted in the body (one of the reactions caused by foreign body contact with the body tissues or fluids) occurs because phagocytes such as macrophages and osteoclasts that adhere to the bone prosthesis identify the bone filler as foreign matter and that the bone prosthesis will not be identified as foreign matter by phagocytes if body fluids are flowing at the site where the phagocytes adhere. Based on this observation, the present inventors proposed that an improved bone prosthesis that will accelerate the calcification of new growth of bone without becoming encapsulated in fibrous tissue can be attained by providing the bone prosthesis with open pores, especially open pores having an average size of 0.01 - 50 $\mu$m. Further, in order to provide greater ease of working for such operations as trimming that are performed during implantation, the mechanical strength of the bone prosthesis having open pores may be lowered by increasing the proportion of the open pores. At the same time, however, the size of the pores is increased and the continuity of the worked surface of the bone prosthesis is decreased to such an extent that it becomes too brittle and friable to be usable as a satisfactory bone prosthesis. As a result of continued studies conducted to solve this problem, the present inventors found that by providing the bone prosthesis with fine closed pores in addition to the open pores, the continuity of the worked surface of the bone prosthesis is retained and its workability can be improved without increasing the average size of the pores. That is, the cross-sectional area of the bone prosthesis is increased without increasing the average size of the pores. The present invention has been accomplished on the basis of this finding.

According to the present invention, the average cross-sectional diameter of the open pores in the bone prosthesis of the present invention is not limited to the range of 0.01 -50 $\mu$m which is specified in the prior invention to be the effective range for the purpose of preventing encapsulation of the bone prosthesis by the fibrous tissue. Pore sizes in this range are particularly preferable in the case where the bone prosthesis is of a comparatively small volume such as in the form of granules, but it has been found that in the case of a bulky bone prosthesis. open pores having a size within this range neither contribute to enhanced flowability of the body fluids nor provide a sufficient number of sites for bone formation to ensure rapid combination with a new growth of bone. Generally speaking, according to the present invention, open pores having an average size in the range of 0.01 - 2,000 $\mu$m provide an adequate number of sites for bone formation and prevent encapsultion of the bone prosthesisby fibrous tissue.

The closed pores in the bone prosthesis of the present invention have an average size in the range of 0.01 -30 $\mu$m, preferably 0.01 - 20 $\mu$m. Closed and discontinuous pores having a size of less than 0.01 $\mu$m are difficult to produce, and pores exceeding 30 $\mu$m in size can lower the strength of the bone prosthesis.

In a preferred embodiment of the present invention, the overall porosity of the bone prosthesis as provided by the combination of the open pores and closed pores is adjusted to be from 5 and 75%. If the overall porosity is less than 5%, the bone prosthesis does not have adequate workability, and if the overall porosity exceeds 75%, the bone prosthesis becomes friable as a result of lowered strength.

The open pores preferably account for 10.0 - 99.6% of all the pores present in the bone prosthesis. If the proportion of the open pores is less than 10.0%, the flowability of the body fluids will be impaired, while if their proportion exceeds 99.6%, little if any effect will be exhibited by the closed pores.

The bone prosthesis of the present invention may be composed of any known type of calcium phosphate based materials, and hydroxyapatite and tri calcium phosphate are particularly preferred.

The bone prosthesis of the present invention may be produced by slight modifications of the methods that are known in the art for producing bone prosthesis having open pores, which are typically classified into the following four techniques: (1) a method wherein a slurry of calcium phosphate based feed material is foamed in the presence of a blowing agent such as aqueous hydrogen peroxide or ovalbumin, and subsequently dried and fired at 900 - 1,400°C; (2) a method wherein a slurry of calcium phosphate based feed material is mixed with a heat-decomposable material having open pores in such a way that the particles in the slurry will be deposited on the heat-decomposable material, and the mixture is fired at 900 - 1,400°C; (3) a method wherein a powder of calcium phosphate based feed material is mixed with beads of a heat-decomposable material, the mixture is shaped with a suitable means such as a press, and then fired at 900 - 1,400°C; and (4) a method wherein a powder of calcium phosphate based feed material is granulated with a suitable granulator such as a drum granulator, and the resulting granules are bound together with an organic binder such as polyvinyl alcohol, and optionally fired at 900 - 1,400°C.

Closed pores within the scope of the present invention may be formed by modifying any of the four methods described above, and in consideration of the manufacturing cost and production rate, such closed pores are preferably formed in the bone prosthesis that is produced by the first method, namely, foaming and drying a slurry of calcium phosphate based feed material. An embodiment of the first method that is modified to form closed pores consists of adding the beads of a heat-decomposable material to the slurry of a calcium phosphate based feed material which is to be foamed with a blowing agent. For instance, heat-decomposable beads having an average size of 0.02 - 40 μm, preferably 0.02 - 30 μm, are added to a slurry of a calcium phosphate based feed material in an amount of 0.1 - 30 wt% of the feed material, and the resulting mixture is foamed in the presence of a blowing agent such as aqueous hydrogen peroxide or ovalbumin, and subsequently dried and fired to form the desired bone prosthesis having both open and closed pores. If the amount of the heat-decomposable material added is less than 0.1 wt% of the calcium phosphate based feed material, a bone prosthesis having improved workability cannot be obtained, while if the heat-decomposable material is added in an amount exceeding 30 wt% of the feed material, the resulting bone prosthesis is too weak to be useful in practical applications. Illustrative heat-decomposable materials that can be added include the beads of synthetic resins such as polystyrene, polyvinyl alcohol and polypropylene, and shreads of cellulose, animal fibers, plant fibers, etc. The firing conditions are in no way limited but, in a typical case, the slurry of calcium phosphate based feed material containing a heat-decomposable material is first heated from room temperature to about 600°C at a rate of 2 - 50°C/hr so as to burn away the heat-decomposable material, and is then heated from 900°C to 1,400°C at a rate of 100 - 200°C/hr and held at that temperature for about 3-8 hours.

The bone prosthesis of the present invention may be used in the following manner: it is first sterilized by a suitable means such as autoclaving, then machined into a predetermined shape with a suitable device such as a sterilized diamond bur under flowing distilled water or physiological saline, and applied to fill a bone defect.

The present invention will now be described by means of the following Example which is not meant to be limiting.

Unless otherwise specified, all percents, ratios, etc., are by weight.

## EXAMPLE

One hundred grams of a hydroxyapatite powder (particle size, 1 - 20 μm) prepared by a known wet process, 5 g of polystyrene beads ("Fine Pearl" manufactured by Sumitomo Chemical Co., Ltd.) having an average size of 6 μm, 5 g of aqueous hydrogen peroxide (product of Wako Pure Chemical Industries, Ltd.; 30% solution) and 160 g of pure water were placed in a polyethylene bag and mixed well by hand. The resulting slurry was transferred into a 200-cc glass beaker, which was set in a forced-air thermostatic dryer and left to stand at 110°C for 24 hours, thereby foaming and drying the slurry. The dried product was recovered from the beaker and transferred to an electric furnace, in which the product was heated from room temperature to 600°C at a rate of 50°C/hr, then form 600°C to 1,200°C at a rate of 100°C/hr. After being held at 1,200°C for 3 hours, the fired product was furnace-cooled to room temperature. The resulting porous sintered hydroxyapatite was cut into cubes. The cubic bone prosthesis thus obtained had the characteristics shown in the Table below.

## TABLE

| Weight ratio of hydroxy- apatite to polystyrene beads | Size (mm) | Porosity (%) | Open pore size (μm) | Closed pore size (μm) | Three- point bending strength (kg/cm²) |
|---|---|---|---|---|---|
| 100/5 | 7x21x31 | 66 | 0.01- 2,000 | 3-6 | 26.8 |

As described in the foregoing, the bone prosthesis of the present invention is composed of a calcium phosphate based porous material having open pores with an average size of 0.01-2,000 μm and closed pores with an average size of 0.01-30 μm. This bone prosthesis-has a satisfactory mechanical strength and yet it can be easily trimmed before and during and a surgical operation for implantation. In addition, after this bone prosthesis has been used to fill a defect in the bone it permits the body fluids to flow in a sufficient amount at the site where phagocytes such as macrophages adhere to ensure that it will not be identified as foreign matter by the phagocytes and thus become encapsulated in fibrous tissue. At the same time, this bone prosthesis provides a sufficient number of sites for bone formation to ensure assimilation by new bone. Therefore, the bone prosthesis of the present invention has the advantages of both good workability and an adequate degree of biocompatibility.

While the invention has been described in detail and with reference to specific embodiments thereof, it will be apparent to one skilled in the art that various changes and modifications can be made therein without departing from the spirit and scope thereof.

### Claims

1. A porous calcium phosphate based bone prosthesis having open pores with an average size of 0.01-2,000 μm and closed pores with an average size of 0.01-30 μm.

2. The porous calcium phosphate based bone prosthesis of Claim 1, wherein the closed pores have an average size of 0.01-20μm.

3. The porous calcium phosphate based bone prosthesis of Claim 1, having a porosity of 5-75%.

4. The porous calcium phosphate based bone prosthesis of Claim 1, wherein the open pores comprise from 10.0-99.6% of all the pores present.

5. The porous calcium phosphate based bone prosthesis of Claim 1, wherein the calcium phosphate based material is one member selected from the group consisting of hydroxyapatite and tri calcium phosphate.